# EUROPEAN PATENT APPLICATION

(11) **EP 1 943 988 A1**
(43) Date of publication of application: **16.07.2008**
(21) Application number: 08000586.1
(22) Date of filing: 14.01.2008
(51) Int. Cl.: A61F 2/84, A61B 19/00, A61B 5/107, A61F 2/00

(54) **Prosthesis deployment apparatus and methods**

(30) Priority: 12.01.2007 US 622943
(71) Applicant: Medtronic Vascular, Inc., Santa Rosa, CA 95403 (US)
(72) Inventor: Yamasaki, Dwayne S., Rohnert Park CA 94928 (US); Bzostek, Andrew, Louisville, CO 80027 (US); Mciff, Greg, Santa Rosa CA 95409 (US)
(74) Representative: Zimmermann, Gerd Heinrich

(57) **Abstract**

The position of a prosthesis (e.g., a stent-graft) is monitored using non-ionizing energy during deployment and/or sheath pull-back after the prosthesis has been positioned at a desired location in a vessel, i.e. using a sensor like an electromagnetic sensing coil, an optical LED, a radio frequency beacon, a pressure wave beacon, an optical reflector, an ultrasound beacon.

## Description

### FIELD OF THE INVENTION

The invention relates to prosthesis deployment and more particularly to monitoring prosthesis position during deployment.

### BACKGROUND OF THE INVENTION

Tubular prostheses such as stents, grafts, and stent-grafts (e.g., stents having an inner and/or outer covering comprising graft material and which may be referred to as covered stents) have been widely used in treating abnormalities in passageways in the human body. In vascular applications, these devices often are used to replace or bypass occluded, diseased or damaged blood vessels such as stenotic or aneurysmal vessels. For example, it is well known to use stent-grafts, which comprise biocompatible graft material (e.g., Dacron ® or expanded polytetrafluoroethylene (ePTFE)) supported by a framework (e.g., one or more stent or stent-like structures), to treat or isolate aneurysms. The framework provides mechanical support and the graft material or liner provides a blood barrier. The graft material for any of the prostheses described herein also can be any suitable material such as Dacron ® material or expanded polytetrafluoroethylene (ePTFE).

Aneurysms generally involve abnormal widening of a duct or canal such as a blood vessel and generally appear in the form of a sac formed by the abnormal dilation of the duct or vessel. The abnormally dilated vessel has a wall that typically is weakened and susceptible to rupture. Aneurysms can occur in blood vessels such as in the abdominal aorta where the aneurysm generally extends below the renal arteries distally to or toward the iliac arteries.

In treating an aneurysm with a stent-graft, the stent-graft typically is placed so that one end of the stent-graft is situated proximally or upstream of the diseased portion of the vessel and the other end of the stent-graft is situated distally or downstream of the diseased portion of the vessel. In this manner, the stent-graft spans across and extends through the aneurysmal sac and beyond the proximal and distal ends thereof to replace or bypass the weakened portion. The graft material typically forms a blood impervious lumen to facilitate endovascular exclusion of the aneurysm.

Such prostheses can be implanted in an open surgical procedure or with a minimally invasive endovascular approach. Minimally invasive endovascular stent-graft use is preferred by many physicians over traditional open surgery techniques where the diseased vessel is surgically opened, and a graft is sutured into position bypassing the aneurysm. The endovascular approach, which has been used to deliver stents, grafts, and stent grafts, generally involves cutting through the skin to access a lumen of the vasculature. Alternatively, lumenar or vascular access may be achieved percutaneously via successive dilation at a less traumatic entry point. Once access is achieved, the stent-graft can be routed through the vasculature to the target site. For example, a stent-graft delivery catheter loaded with a stent-graft can be percutaneously introduced into the vasculature (e.g., into a femoral artery) and the stent-graft delivered endovascularly to a portion where it spans across the aneurysm where it is deployed.

When using a balloon expandable stent-graft, balloon catheters generally are used to expand the stent-graft after it is positioned at the target site. When, however, a self-expanding stent-graft is used, the stent-graft generally is radially compressed or folded and placed at the distal end of a sheath or delivery catheter and self expands upon retraction or removal of the sheath at the target site. More specifically, a delivery catheter having coaxial inner and outer tubes arranged for relative axial movement therebetween can be used and loaded with a compressed self-expanding stent-graft. The stent-graft is positioned within the distal end of the outer tube (sheath) and in front of a stop fixed to distal end of the inner tube. Regarding proximal and distal positions referenced herein, the proximal end of a prosthesis (e.g., stent-graft) is the end closest to the heart (by way of blood flow) whereas the distal end is the end furthest away from the heart during deployment. In contrast, the distal end of a catheter is usually identified as the end that is farthest from the operator, while the proximal end of the catheter is the end nearest the operator. Once the catheter is positioned for deployment of the stent-graft at the target site, the inner tube is held stationary and the outer tube (sheath) withdrawn so that the stent-graft is gradually exposed and expands. An exemplary stent-graft delivery system is described in U.S. Patent Application Publication No. 2004/0093063, which published on May 13, 2004 to Wright et al. and is entitled Controlled Deployment Delivery System, the disclosure of which is hereby incorporated herein in its entirety by reference.

Although the endovascular approach is much less invasive, and usually requires less recovery time and involves less risk of complication as compared to open surgery, there can be concerns with alignment of asymmetric features of various prostheses in relatively complex applications such as one involving branch vessels. Branch vessel techniques have involved the delivery of a main device (e.g., a graft or stent-graft) and then a secondary device (e.g., a branch graft or branch stent-graft) through a fenestration or side opening in the main device and into a branch vessel.

The procedure becomes more complicated when more than one branch vessel is treated. One example is when an aortic abdominal aneurysm is to be treated and its proximal neck is diseased or damaged to the extent that it cannot support a reliable connection with a prosthesis. In this case, grafts or stent-grafts have been provided with fenestrations or openings formed in their side wall below a proximal portion thereof. The fenestrations or openings are to be aligned with the renal arteries and the proximal portion is secured to the aortic wall above the renal arteries.

To ensure alignment of the prostheses fenestrations and branch vessels, some current techniques involve placing guidewires through each fenestration and branch vessel (e.g., artery) prior to releasing the main device or prosthesis. This involves manipulation of multiple wires in the aorta at the same time, while the delivery system and stent-graft are still in the aorta. In addition, an angiographic catheter, which may have been used to provide detection of the branch vessels and preliminary prosthesis positioning, may still be in the aorta. Not only is there risk of entanglement of these components, the openings in an off the shelf prosthesis with preformed fenestrations may not properly align with the branch vessels due to differences in anatomy from one patient to another. Prostheses having preformed custom located fenestrations or openings based on a patient's CAT scans also are not free from risk. A custom designed prosthesis is constructed based on a surgeon's interpretation of the scan and still may not result in the desired anatomical fit. Further, relatively stiff catheters are used to deliver grafts and stent-grafts and these catheters can apply force to tortuous vessel walls to reshape the vessel (e.g., artery) in which they are introduced. When the vessel is reshaped, even a custom designed prosthesis may not properly align with the branch vessels.

Self-expanding stent-grafts require withdrawal of restrictive sheath during deployment as discussed above. That is, the stent-graft is allowed to open through the removal of the sheath. The sheath is typically removed using a pull-back motion. One challenge with such a pull back approach is that the catheter or stent-graft itself may move when the sheath is retracted. If the catheter is moved during sheath retraction, then the stent-graft may not be implanted in its desired location. As such, physicians are always concerned with ensuring the catheter and/or stent-graft remains properly positioned during sheath pullback. Generally speaking, physicians often use fluoroscopic imaging techniques to confirm that the catheter and or stent-graft remains properly positioned during deployment. This approach requires one to administer a radiopaque substance, which generally is referred to as a contrast medium, agent or dye, into the patient so that it reaches the area to be visualized (e.g., the renal arteries). A catheter can be introduced through the femoral artery in the groin of the patient and endovascularly advanced to the vicinity of the renals. The fluoroscopic images of the transient contrast agent in the blood, which can be still images or real-time motion images, allow two dimensional visualization of the location of the renals.

The use of X-rays, however, requires that the potential risks from a procedure be carefully balanced with the benefits of the procedure to the patient. While physicians always try to use low dose rates during fluoroscopy, the duration of a procedure may be such that it results in a relatively high absorbed dose to the patient and physician. Patients who cannot tolerate contrast enhanced imaging or physicians who must or wish to reduce radiation exposure need an alternative approach for defining the vessel configuration and branch vessel location.

Accordingly, there remains a need to develop and/or improve prosthesis deployment apparatus and methods for endoluminal or endovascular applications.

### SUMMARY OF THE INVENTION

The present invention involves improvements in prosthesis deployment apparatus and methods.

In one embodiment according to the invention, a method of monitoring prosthesis deployment in a patient comprises positioning a prosthesis delivery catheter, having a proximal end portion and a distal end portion, and a prosthesis having a proximal end portion and a distal end portion disposed in the catheter distal end portion and at least one marker adapted to generate, respond to, or modify nonionizing energy, nonionizing radiation, or nonionizing fields, the marker being on one of the prosthesis and prosthesis delivery catheter with the prosthesis proximal end portion at a desired location in the vasculature of the patient; deploying the prosthesis from the catheter; and monitoring prosthesis movement based on the marker's generation of, response to, or modification of nonionizing energy, nonionizing radiation, or nonionizing fields.

In a further embodiment, a method of monitoring prosthesis deployment in a patient comprises positioning a prosthesis delivery catheter, having a proximal end portion and a distal end portion, and a prosthesis having a proximal end portion and a distal end portion disposed in the catheter distal end portion and at least one marker provided on one of the prosthesis and prosthesis delivery catheter with the prosthesis proximal end portion at a desired location in the vasculature of the patient; subjecting the at least one marker to electromagnetic energy; deploying the prosthesis from the catheter; and monitoring prosthesis movement based on a reaction that results from subjecting the at least one marker to the electromagnetic energy.

In another embodiment according to the invention, a method of monitoring prosthesis deployment in a patient comprises positioning a prosthesis delivery catheter, having a proximal end portion and a distal end portion, and a prosthesis having a proximal end portion and a distal end portion disposed in the catheter distal end portion and at least one electromagnetic field generating marker provided on one of the prosthesis and prosthesis delivery catheter with the prosthesis proximal end portion at a desired location in the vasculature of the patient; deploying the prosthesis from the catheter; and monitoring prosthesis movement based on the electromagnetic field generated by the at least one marker in response to electromagnetic field or a radiofrequency field.

In another embodiment according to the invention, a method of monitoring prosthesis deployment in a patient comprises positioning a prosthesis delivery catheter, having a proximal end portion and a distal end portion, and a prosthesis having a proximal end portion and a distal end portion disposed in the catheter distal end portion and at least one non-ionizing photon radiation marker provided on one of the prosthesis and prosthesis delivery catheter with the prosthesis proximal end portion at a desired location in the vasculature of the patient; deploying the prosthesis from the catheter; and monitoring prosthesis movement based on generation of non-ionizing radiation generated by the at least one marker in response to exposure to energy frequecies of the electromagnetic spectrum such as ultraviolet or infared.

In another embodiment according to the invention, a method of monitoring prosthesis deployment in a patient comprises positioning a prosthesis delivery catheter, having a proximal end portion and a distal end portion, and a prosthesis having a proximal end portion and a distal end portion disposed in the catheter distal end portion and at least one marker adapted to modify non-ionizing photon radiation provided on one of the prosthesis and prosthesis delivery catheter with the prosthesis proximal end portion at a desired location in the vasculature of the patient; subjecting the marker to non-ionizing photon radiation; deploying the prosthesis from the catheter; and monitoring prosthesis movement based on the at least one marker's modification of the non-ionizing photon radiation.

In another embodiment according to the invention, a method of monitoring prosthesis deployment in a patient comprises positioning a prosthesis delivery catheter, having a proximal end portion and a distal end portion, and a prosthesis having a proximal end portion and a distal end portion disposed in the catheter distal end portion and at least one marker adapted to generate pressure waves provided on one of the prosthesis and prosthesis delivery catheter with the prosthesis proximal end portion at a desired location in the vasculature of the patient; energizing the marker with ultrasound generated from transducers deploying the prosthesis from the catheter; and monitoring prosthesis movement based on pressure waves generated at the least one marker when energized.

In another embodiment according to the invention, a prosthesis delivery system comprises a tubular sheath having a proximal end portion and a distal end portion; a distal tip extending from the tubular sheath distal end portion; a tubular prosthesis slidably disposed in the sheath and positioned along the sheath distal end portion; and a marker provided on the prosthesis and adapted to provide measurable data when subjected to non-ionizing energy.

Other features, advantages, and embodiments according to the invention will be apparent to those skilled in the art from the following description and accompanying drawings.

### BRIEF DESCRIPTION OF THE DRAWINGS

FIG. 1 diagrammatically illustrates one embodiment of a prosthesis delivery system in accordance with the invention.

FIG. 1A is a sectional view taken along line 1A-1A in FIG. 1.

FIG. 2A illustrates one embodiment of prosthesis delivery system of FIG. 1 in a loaded state.

FIG. 2B illustrates the prosthesis delivery system of FIG. 2A in a partially deployed state delivery system of FIG. 1 coupled to the circuit of FIG. 2.

FIG. 2C illustrates the prosthesis delivery system of FIG. 2B with the prosthesis proximal end deployed.

FIG. 3A diagrammatically illustrates one prosthesis-location device combination according to the invention.

FIG. 3B is a view of the prosthesis-location device combination of FIG. 3A taken along line FIG. 3B-3B.

FIG. 4 diagrammatically illustrates another prosthesis-location device combination according to the invention.

FIG. 5 diagrammatically illustrates another prosthesis-location device combination according to the invention.

FIG. 6 illustrates one embodiment of a device location system according to the invention incorporating the prosthesis delivery system of FIG. 1.

FIGS. 7-9 depict one embodiment of a method according to the invention where FIG. 7 depicts endovascular prosthesis delivery to a target site using the system of FIG. 1; FIG. 8 depicts partial sheath pull-back while monitoring the position of the prosthesis; and FIG. 9 depicts the prosthesis fully deployed and a contralateral leg secured thereto.

FIG. 10A diagrammatically illustrates a known system for energizing and locating leadless electromagnetic markers.

FIG. 10B is a schematic isometric view of the receiver of FIG. 10A.

FIG. 10C is a diagrammatical section view of a known leadless electromagnetic marker.

### DETAILED DESCRIPTION

The following description will be made with reference to the drawings where when referring to the various figures, it should be understood that like numerals or characters indicate like elements.

Regarding proximal and distal positions, the proximal end of the prosthesis (e.g., stent-graft) is the end closest to the heart (by way of blood flow) whereas the distal end is the end farthest away from the heart during deployment. In contrast, the distal end of the catheter is usually identified as the end that is farthest from the operator, while the proximal end of the catheter is the end nearest the operator. Therefore, the prosthesis (e.g., stent-graft) and delivery system proximal and distal descriptions may be consistent or opposite to one another depending on prosthesis (e.g., stent-graft) location in relation to the catheter delivery path.

Embodiments according to the invention facilitate monitoring the position of a prosthesis (e.g., a stent-graft) in real time during deployment and/or sheath pull-back after the prosthesis has been positioned at a desired location in a vessel. The target site can be at various locations along the vessel including locations adjacent to a branch vessel. Branch lumens emanate from the intersection of a vessel (e.g., the aorta) and other attendant vessels (e.g., major arteries such as the renal, brachiocephalic, subclavian and carotid arteries, and minor arteries such as the intercostals and lumbar arteries).

According to one embodiment of the invention, one or more markers or locating devices are positioned on or integrally formed in the distal tip portion of a prosthesis delivery catheter to provide information indicative of prosthesis movement during catheter sheath pull-back. In an alternative configuration, one or more locating devices are positioned on or integrally formed in the prosthesis, which can be in the form of a stent-graft, to provide information regarding prosthesis movement during prosthesis deployment from the prosthesis delivery catheter. The prosthesis movement is monitored based on the marker's generation of, response to, or modification of nonionizing energy, nonionizing radiation, or nonionizing fields.

In contrast to the embodiments described above, PET, SPECT, Fluoroscopic, and CT location markers operate in ionizing energy systems (photon radiation -ionizing systems). PET and SPECT involve radioactive components that are injected into the patient and can be referred to as active components in that they sense energy. Markers used in fluoroscopy and CT procedures can be referred to as passive in that they block or modify the ionizing energy.

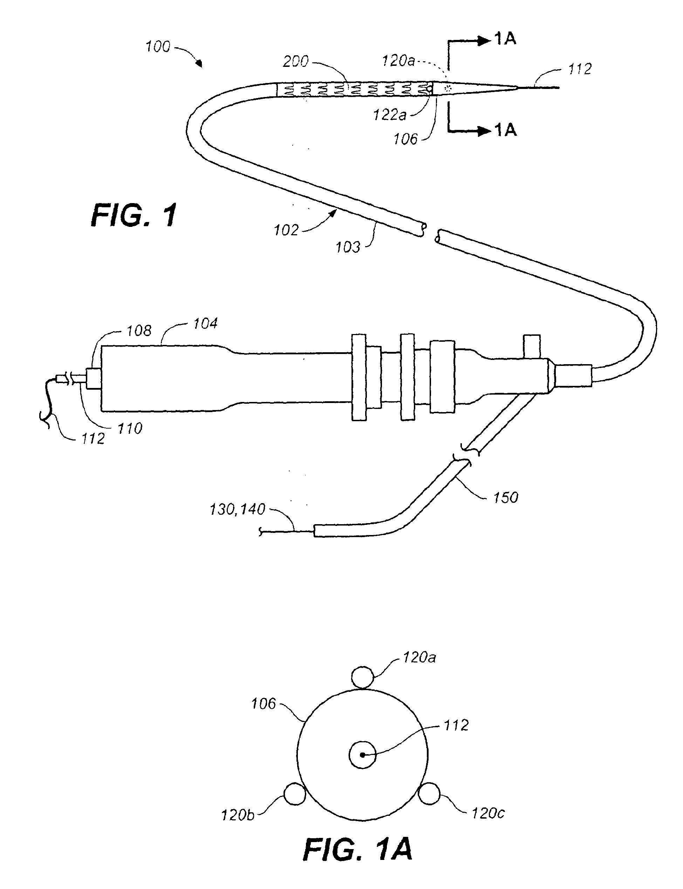

Referring to FIG. 1, one embodiment of a prosthesis delivery system according to the invention is shown and generally designated with reference numeral 100. Prosthesis delivery system 100 comprises catheter 102, control handle 104, flexible tapered tip member (or obturator 106), which can form a portion of the distal end of the catheter. Handle 104 includes an inlet 108, through which central guidewire lumen 110 enters the handle and extends to flexible tapered tip member 106, which has an axial bore for slidably receiving guidewire 112. Tapered tip member 106 is placed at the distal end of catheter sheath 103 and handle 104 is affixed to the proximal end of catheter sheath 103. A guidewire 112 can be slidably disposed in guidewire lumen 110 and catheter 112 tracked thereover. When the prosthesis to be delivered is a self-expanding graft or stent-graft (such as stent-graft 200), it generally is radially compressed or folded and placed in the distal end portion of the delivery catheter and allowed to expand upon deployment from the catheter at the target site as will be described in detail below. Stent-graft 200 can include a plurality of undulating stent elements to support the tubular graft material as is known in the art.

In the illustrative embodiment, various marker configurations are depicted. According to one variation, one or more markers 120a, 120b, 120c...120n are secured to or integrally formed in tapered tip 106. In one example of this variation shown in FIG. 1A, three markers 120a,b,c are attached or formed in tapered tip 106 and approximately equidistantly spaced apart in the circumferential direction (i.e., spaced about 120 degrees from one another). In another variation, one or more markers 122a, 122b, 122c...122n are secured to or integrally formed in prosthesis 200 in a proximal portion thereof. These markers also can be approximately equidistantly spaced from one another in a circumferential direction. In yet another variation, one or more of the aforementioned non-ionizing markers can be provided in both the tapered tip and prosthesis.

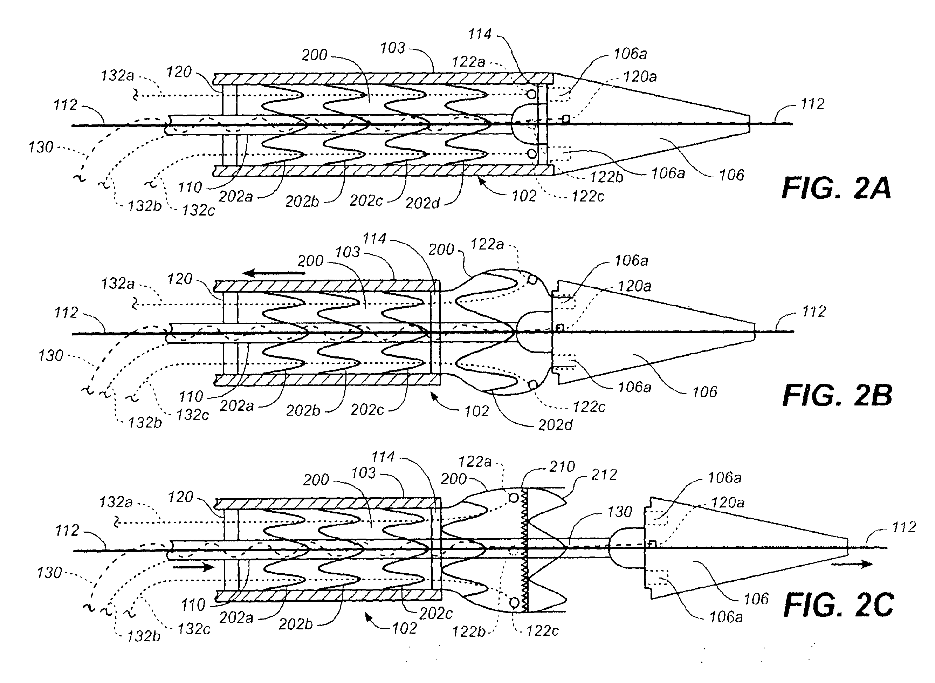

Referring to FIGS. 2A-C, one delivery catheter system configuration according to the invention is shown in a pre-deployment loaded state FIG. 2A and two partial deployment states (FIG. 2B & 2C). Delivery catheter 102 includes catheter sheath 103, which can be referred to as an outer tube, and inner guidewire tube 110. Sheath 103 and guidewire tube 110 are coaxial and arranged for relative axial movement therebetween. The prosthesis (e.g., stent-graft 200) is positioned within the distal end of outer tube 103 and in front of pusher member or stop 120, which is concentric with and secured to inner guidewire tube 110 and can have a disk or ring shaped configuration with a central access bore to provide access for guidewire tube 110. In the example where prosthesis 200 comprises a stent-graft as shown in the illustrative embodiment, the stent graft comprises a tubular graft member and a plurality of annular undulated stent elements, such as stent elements 202a,b,c,d, to provide structural support to the graft as is known in the art. An undulating bare spring element 212 also can be sutured or otherwise attached to the proximal end of the prosthesis and/or an annular undulating wire 210 having an undulating configuration secured to the proximal end of the prosthesis to provide radial strength as well. The spring has a radially outward bias so that when it is released from a radially collapsed or restrained state it expands outwardly to secure the proximal portion of the prosthesis to the target passageway wall. Another undulating wire 210 can be attached to the prosthesis distal end as well or in the alternative. More specifically, a support spring 210 can be provided at one or both ends of the prosthesis. The stent and support elements can be positioned on the interior and/or exterior of the graft member and secured thereto by suturing or other conventional means.

A radiopaque ring 114 can be provided on the inside of the distal end portion of sheath 103 in overlapping relation to the tapered tip (FIG. 2A) to assist with imaging distal end of sheath 103 using fluoroscopic techniques. Alternatively, radiopaque ring 114 can be provided on the proximal end of the tapered tip. Once the catheter is positioned for deployment of the prosthesis at the desired site, the inner member or guidewire lumen 110 with stop 120 are held stationary and the outer tube or sheath 103 withdrawn so that tapered tip 106 is displaced from sheath 103 and the stent-graft gradually exposed and allowed to expand. Stop 120 therefore is sized to engage the distal end of the stent-graft as the stent-graft is deployed. The proximal ends of the sheath 103 and inner tube or guidewire lumen 110 are coupled to and manipulated by handle 104. Tapered tip 106 optionally can be configured with an annular recess or cavity 106a formed in its distal end portion and configured to receive and retain the proximal end portion of the prosthesis in a radially compressed configuration before allowing expansion thereof during a later phase of its deployment. Alternatively, any of the stent-graft deployment systems described in U.S. Patent Application Publication No. 2004/0093063, which published on May 13, 2004 to Wright et al. and is entitled Controlled Deployment Delivery System, the disclosure of which is hereby incorporated herein by reference in its entirety, can be incorporated into stent-graft delivery system 100.

Referring to FIG. 2B, the prosthesis delivery is shown with catheter sheath 103 partially pulled back and a portion of the prosthesis partially expanded. In this partially retracted position, the proximal end of the prosthesis is constrained allowing the prosthesis to be repositioned (e.g., longitudinally or rotationally moved) if desired before release of the proximal end of the prosthesis. The surgeon can determine if prosthesis repositioning is desired based on monitored movement of the prosthesis during deployment as will be described in more detail below.

Referring to FIG. 2C, the catheter sheath is held stationary and guide lumen 110, which is fixedly secured to tapered tip 106, is advanced to separate tapered tip 106 from catheter sheath 103 and release the proximal end of prosthesis 200.

Depending on whether the markers are leadless or wired, markers 120a,b,c can have separate leads which are bundled in cable 130. Cable 130 can extend though guidewire tube 110 and out from a slot formed therein into sleeve 150 to an energy source or measuring circuit as will be described below. Similarly markers 122a,b,c can have separate leads 132,a,b,c, which extend between the prosthesis and catheter sheath or between the prosthesis and guidewire tube 110 and are bundled in cable 140, which extends into sleeve 150 to an energy source or measuring circuit.

The non-ionizing energy target localization system used in conjunction with the markers can be electromagnetic field based, photon-radiation based, or pressure wave (e.g., ultrasound) based. The markers or location components can be active or passive depending on their type and the non-ionizing energy based localization system or approach used. Markers or components that generate or sense energy when used with a target localization system can be referred to as active markers or components, while markers or location components that block or modify energy when used with a target localization system can be referred to as passive markers or components.

When using electromagnetic radiation, the markers can be wired active devices in the form of magnetically sensitive, electrically conductive sensing coils or they can be in the form of lead less passive devices, which can be in the form of AC electromagnetic devices.

When magnetically sensitive, electrically conductive sensing coils are used, the coil positions can be located by determining the positions of the coils relative to a plurality of magnetic field sources of known location. Pre-specified electromagnetic fields are projected to the portion of the anatomical structure of interest (e.g., that portion that includes all prospective locations of the coils in a manner and sufficient to induce voltage signals in the coil(s). Electrical measurements of the voltage signals are made to compute the angular orientation and positional coordinates of the sensing coil(s) and hence the location of the vasculature and/or devices of interest. An example of sensing coils having signal transmitting leads to provide information for determining the location of a catheter or endoscopic probe inserted into a selected body cavity of a patient undergoing surgery in response to prespecified electromagnetic fields is disclosed in U.S. Patent No. 5,592,939 to Martinelli, the disclosure of which is hereby incorporated herein by reference in its entirety. Another example of methods and apparatus for locating the position in three dimensions of a sensor comprising a sensing coil by generating magnetic fields which are detected at the sensor is disclosed in U.S. Patent No. 5,913,820 to Bladen, et al., the disclosure of which is hereby incorporated herein by reference in its entirety. The Stealth Station®AXIEM™ electromagnetic technology is an example of an electromagnetic guided platform and is available from Medtronic, Inc. (Minneapolis, Minn.). Another electromagnetic platform is the Magellan electromagnetic navigation system (Biosense Webster, Tirat HaCarmel, Israel)

Electromagnetically sensitive leadless coils also can be used to locate a coil type marker in three-dimensional space as will be described in more detail below.

In the case of a non-ionizing photon radiation energy approach, active markers can include optical LEDs or radio frequency (RF) beacons and passive markers can include optical or RF reflectors, as understood an known by persons skilled in the art.

When a non-ionizing pressure wave (e.g., ultrasound) approach is used, active markers such as markers made by U.S. Beacons can be used. Location markers using the ImPressure pressure sensor (Remon Medical Technologies, Caesarea, Israel) can be used. The sensor is a miniaturized device that measures 3 mm x 9 mm x 1.5 mm (Figure 1). It is an ultrasound-based technology that remains quiescent until acoustically activated. Once activated, acoustic energy is converted into electrical energy and a location beacon origin location measurement is transferred to the monitor through acoustic energy. The location of the sensor can therby be precisely mapped.

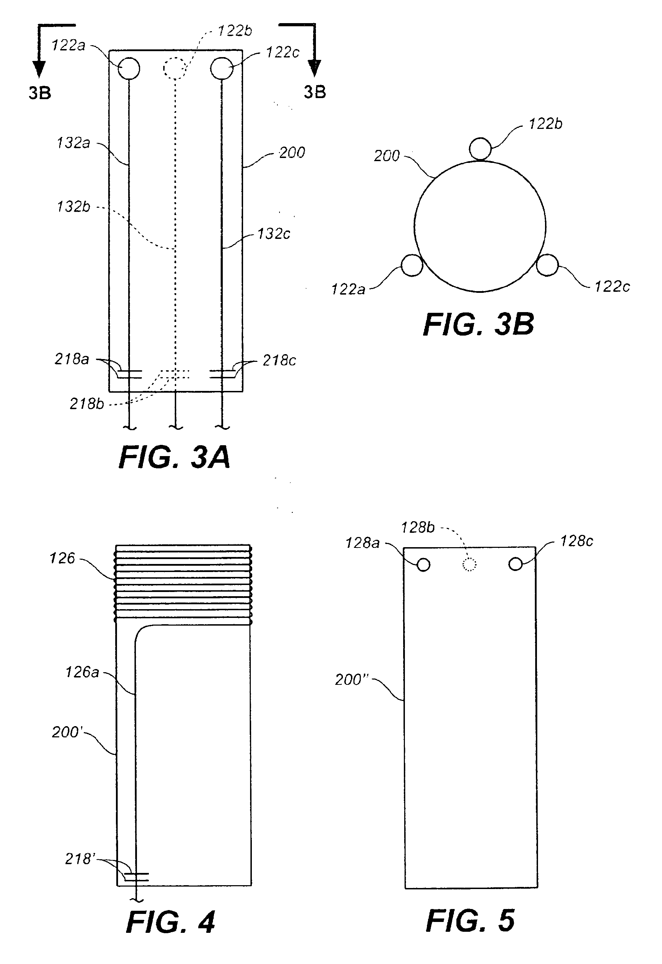

Referring to FIGS. 3A and 3B, a wired embodiment of prosthesis 200 is shown with markers attached to the proximal end of the prosthesis with sutures or other suitable means. Although three markers 122a, 122b, and 122c, which can be electromagnetic sensing coils comprising, for example, stainless steel coated copper, are shown, other multiples of markers can be used. Leads or conductors 132a, 132b, and 132c, which can be in the form of wires, extend respective markers 122a, 122b, and 122c to bundle cable sheath 130 as described above. A portion of each lead or conductor is secured to the distal end of the prosthesis as indicated with reference numerals 218a, 218b, and 218c, which can correspond to sutures or other suitable fasteners.

Referring to FIG. 4, another prosthesis 200' is shown where the marker is an electromagnetic sensing coil that is wound around the proximal end of the prosthesis. This configuration provides a relatively large coil which can be stainless steel. Although sensing coils also can be platinum, platinum iridium, or aluminum, though aluminum typically is not suitable for implant applications. Lead 126a extends from marker 126 for coupling to a processor as will be described in more detail below. A portion of lead or conductor 126a is secured to the distal end of the prosthesis as indicated with reference numeral 218', which can correspond to sutures or other suitable fasteners.

Referring to FIG. 5, another prosthesis embodiment is shown and generally indicated with reference numeral 200". Prosthesis 200" includes one or more leadless markers that are secured to the distal end thereof by suturing or other suitable means. In the illustrative three markers 128a, 128b, and 128, which are approximately equidistantly spaced in the circumferential direction, are shown for purposes of example.

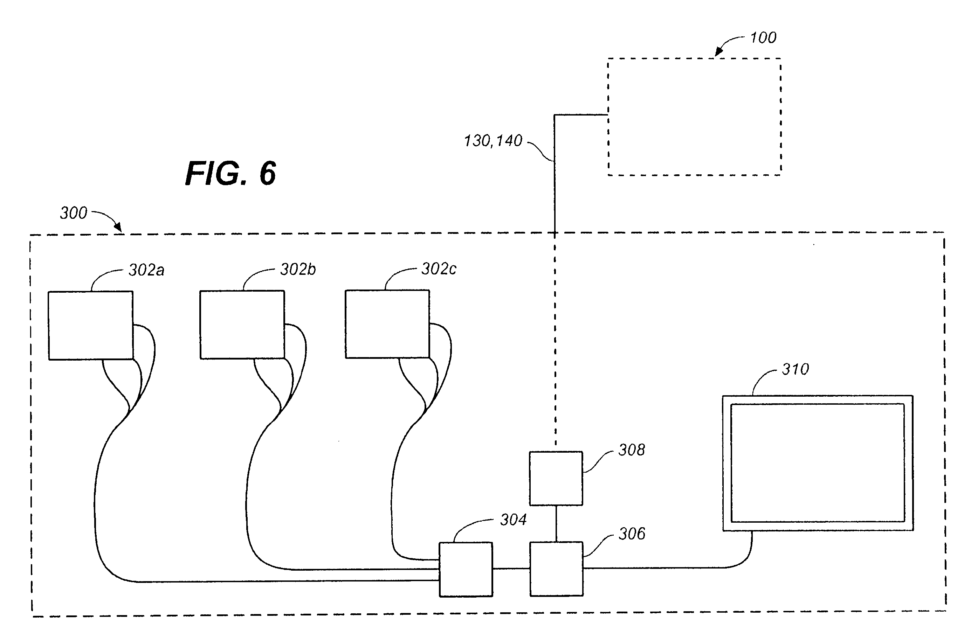

Referring to FIG. 6, one localization system for providing the position or orientation of a target in three dimensions is shown. The field generating and signal processing circuit configuration for generating magnetic fields at the location of the sensors and processing the voltage signals that the sensors generate in response to the generated magnetic fields, when the sensors are conductive sensing coils, is generally designated with reference numeral 300.

Circuit 300 generally includes three electromagnetic field (EMF) generators 302a, 302b, and 302c, amplifier 304, controller 306, measurement unit 308, and display device 310. Each field generator comprises three electrically separate coils of wire (generating coils). The nine generating coils are separately electrically connected to amplifier 304, which drives each coil individually and sequentially in response to the direction of controller 306. Although nine coils are shown in three groups of three in the example depicted in FIG. 6, it should be understood that nine separate coils can be used.

Once the quasi-static field from a particular generating coil is established, measurement unit 308 measures the value of the voltage that the field induces in each sensing coil. This data is processed and passed to controller 306, which stores the value and then instructs amplifier 304 to stop driving the present generating coil and to start driving the next generating coil. When all generating coils have been driven, or energized, and the corresponding nine voltages induced into each sensing coil have been measured and stored, controller 306 calculates the location and orientation of each sensor relative to the field generators and displays this on a display device 310. This calculation can be carried out while the subsequent set of nine measurements are being taken. Thus, by sequentially driving each of the nine generating coils, arranged in three groups of three mutually orthogonal coils, the location and orientation of each sensing coil can be determined.

Any suitable electromagnetic field generating and signal processing circuit for locating sensor position in three dimensions can be used (see e.g., U.S. Patent No. 5,913,820 to Bladen, et al. (*supra*) regarding magnetically sensitive, electrically conductive sensing coils (e.g., antenna coils)). The sensor and generating coil specifications, as well as the processing steps are within the skill of one of ordinary skill of the art. An example of coil specifications and general processing steps that can be used are disclosed in U.S. Patent No. 5,913,820 to Bladen, et al., the disclosure of which is hereby incorporated herein by reference in its entirety.

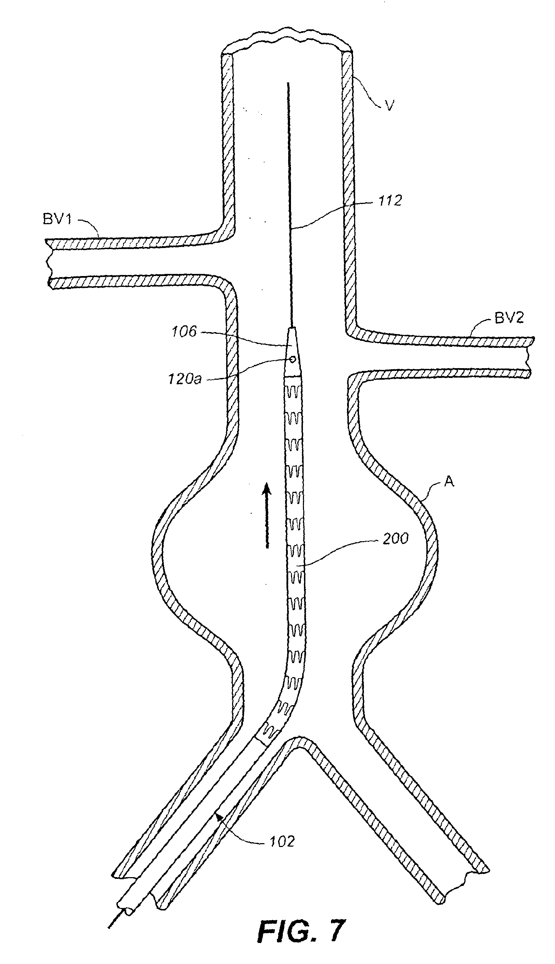

Referring to FIGS. 7-9, an exemplary operation of a system according to the invention will now be described using prosthesis delivery system 100. For the purposes of the example, the procedure involves the endovascular delivery and deployment of an AAA bifurcated stent-graft and an electromagnetic field positioning system including three electromagnetic field sensing coil type sensors attached to catheter tapered tip 106 (two are hidden from view). It should be understood, however, that other sensor multiples can be used. For example, a single sensor can be used.

Regarding registration, numerous methods can be employed. In one example, a preoperative image can be registered via two-dimensional or three-dimensional fluoroscopy. For example, after the preoperative data is acquired, a two-dimensional image is taken intraoperatively and is registered with the preoperative image as is known in the art (see U.S. Patent Publication No. 2004/021571 regarding registering two-dimensional and three-dimensional images. The disclosure of U.S. Patent Publication No. 2004021571 is hereby incorporated herein by reference in its entirety). In another example, an O-arm™ imaging system can be used intraoperatively to take a picture/image of the navigation site to be navigated (see., e.g., U.S. Patent No. 6,940,941, U.S. Patent No. 7,001,045, U.S. Patent Publication No. 2004/013225, U.S. Patent Publication No. 2004/0013239, U.S. Patent Publication No. 2004/0170254, and U.S. Patent Publication No. 2004/0179643, the disclosures of which are hereby incorporated by reference in their entirety. Another representative system that performs image registration is described in U.S. Patent No. 6,470,207 (Simon, et al.), the disclosure of which is hereby incorporated herein by reference in its entirety. In this example, a two-dimensional image will be registered with a three-dimensional image.

Prior to the surgical procedure, the patient is scanned using either a CT, CTA, MRI, or MRA scanner to generate a three-dimensional model of the vasculature to be tracked. The abdominal aorta and branch vessels of interest (e.g., renal arteries) are scanned and images taken therealong to create a three-dimensional pre-procedural data set for that vasculature and create a virtual model upon which real time data will be overlaid. The virtual model is input into software that consolidates all of the navigation information and displays information that the user sees.

The three magnetic field generators 502a, 502b, and 502c are fixed in a predetermined position so that the spatial relationship between the field generator coils and the sensing coils can be determined. Alternatively, nine separate field generating coils can be used instead of magnetic field generators 502a, 502b, and 502c as described above.

The patient is prepared for surgery and a cut is made down to a femoral artery and a guidewire inserted. A device which measures the position of the patient in the magnetic field is placed as a reference, either external to the patient or intravascularly. Anatomical reference point of the patient in the magnetic field are or have been placed as a reference, either external to the patient or intravascularly. Examples of external markers are fiducial markers on the patient's skin over vertebra or on skin over the iliac crest. Examples of internal markers are markers placed in the wall of the renals arteries of other branches of the aorta. A contrast agent catheter is delivered through the femoral artery and the vasculature perfused with contrast and a fluoroscopic image up to the renal arteries taken. The processor orients or registers the intraoperative fluoroscopic X-ray image with the three-dimensional preoperative image as is known in the art. One example is described in U.S. Patent Publication No. 2004/021571 to Frank, et al., the disclosure of which is hereby incorporated herein by reference in its entirety.

Referring to FIG. 7, the operator tracks catheter 102 over guidewire 112 toward aneurysm A and branch vessels BV1 and BV2, which branch from vessel V, which in this example is the aorta. The position of the distal end of catheter sheath 103 is monitored virtually based on the signals received from the sensors on the tapered tip and the known catheter dimensions and position of the sensors relative to distal end of the catheter sheath 103, all of which were entered into the processor, to indirectly provide the position of the proximal end of stent-graft 200. Alternatively, the position of the proximal end of stent-graft 200 is monitored virtually based on the signals received from the sensors on the tapered tip and the known catheter dimensions and position of the sensors relative to proximal end of the stent-graft 200. This information can be entered into the processor in the alternative or in addition to that described above.

The display will show the position of the sensors and/or the catheter sheath distal end as they move toward the lower renal artery. Alternatively, the sensors and/or the stent-graft proximal end can be displayed.

In the vicinity of the target location (e.g., the lower renal artery), which the operator can estimate based on the three-dimensional model and the sensor positions, the operator can use fluoroscopy to position the stent-graft at the desired location below the lower renal ostia. Fluoroscopy also can be helpful in the positioning step when the aorta is very tortuous and the catheter significantly changed the aorta's configuration (the configuration of the virtual model) during advancement therethrough.

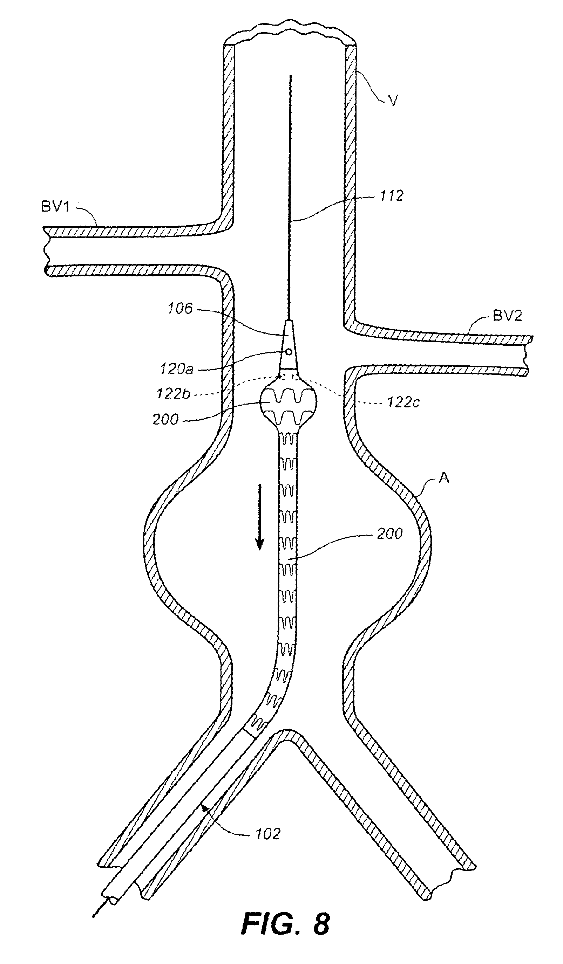

Referring to FIG. 8, once the stent-graft is in the desired position, the operator holds guidewire tube 110 and pusher disk 120 stationary, and retracts or pulls back catheter sheath 103. As the sheath is pulled back, the position of the sensors are monitored to determine if stent graft movement beyond a threshold value occurs. A virtual image of the sensors and stent-graft can be displayed on the monitor so that the surgeon can monitor the sensors qualitatively, either individually or in coordination with two-dimensional or three-dimensional images generated pre-operatively or intraoperatively. Alternatively, the processor can display a warning when the sensors move more than a threshold value (e.g., more than 2mm) in the direction of blood flow or in a direction generally parallel to the central axis of the proximal end portion of the stent-graft. In a further alternative, the distance between the base of the ostium and the sensors, measured in the direction of blood flow or along the aortic wall in a distal direction (away from the heart), is displayed to provide a quantitative value of stent-graft movement in that direction. A non-symmetric marker array pattern will provide a precise incremental image of any rotation or other translation of the prosthesis. In contrast to a symmetrical array whose image would repeat and might be indistinguishable if it were rotated, by exactly one incremental mark. In yet a further alternative, the annular proximal end of the stent-graft can monitored based in stent-graft dimensional data and the position of the sensor relative to the proximal end of the stent-graft, which can be readily input into the processor.

If the position of the stent-graft proximal portion changes more than a desired amount, the operator has a limited opportunity to reposition the stent-graft.

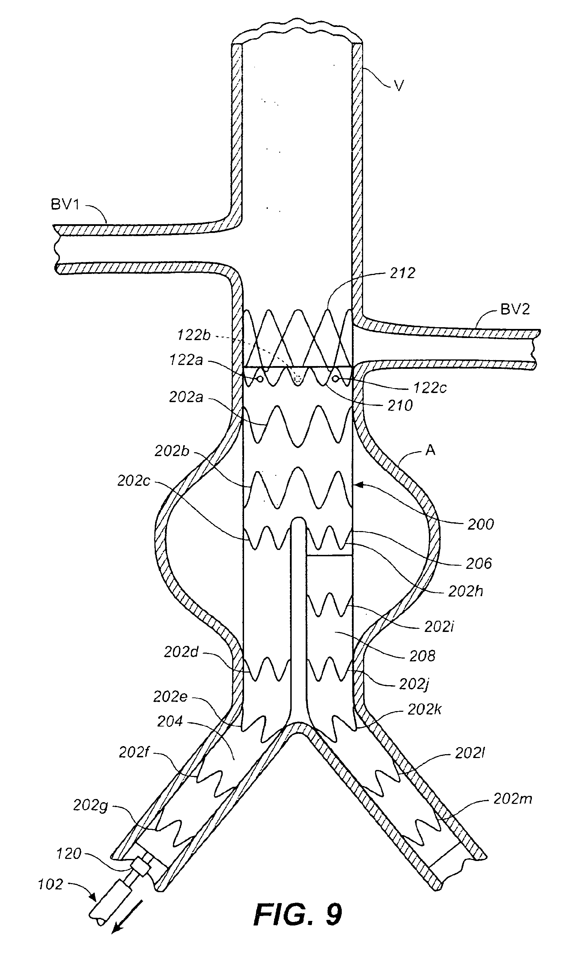

After the stent-graft is deployed at the desired position, all catheters are withdrawn. The fully deployed stent-graft is shown in FIG. 9 illustrating ipsilateral leg 204 and contralateral stump 206 having contralateral leg 208 coupled thereto using conventional techniques. The combined prosthesis includes stent elements 202a-m.

As an alternative to sensors 120a,b,c, sensors 122a,b,c can be used. Sensors 122a,b,c being attached to the proximal end of the stent-graft can provide signals indicative of the position of the stent-graft proximal end or a virtual image of the stent-graft proximal end based on the distance between the sensors and the stent-graft proximal end, which can be input into the processor. In this case, endoscopic instruments can be used to cut leads 132,a,b,c proximal to sutures or fasteners 218a,b,c.

Although an electromagnetic field and sensing coil position type imaging system, which uses non-ionizing radiation energy, has been described in the illustrative embodiment, other non-ionizing energy platforms or localization systems can be used as described above.

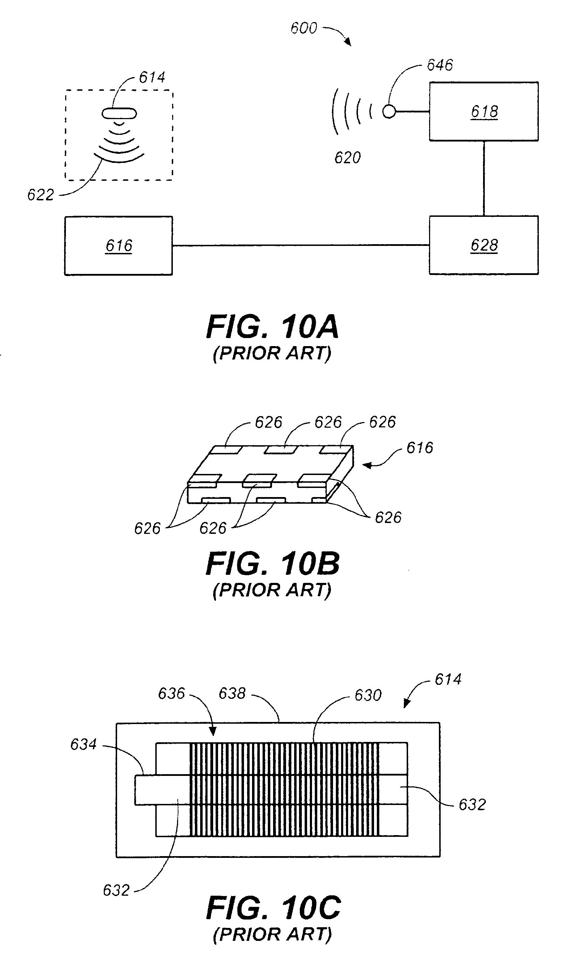

FIGS. 10A-C illustrate a system and components for generating an excitation signal for activating a leadless resonating marker assembly and locating the marker in three-dimensional space which can be used in systems for performing methods in accordance with aspects of the present invention.

Referring to FIGS. 10A-C, a known leadless electromagnetic system is shown. FIG. 10A is a schematic view of a system 600 for energizing and locating one or more leadless resonating marker assemblies 614 in three-dimensional space relative to a sensor array 616 where one marker assembly 614 is shown in this example. System 600 includes a source generator 618 that generates a selected magnetic excitation field or excitation signal 620 that energizes each marker assembly 614. Each energized marker assembly 614 generates a measurable marker signal 622 that can be sufficiently measured in the presence of both the excitation source signal and environmental noise sources. The marker assemblies 614 can be positioned in or on a selected object in a known orientation relative to each other. The marker signals 622 are measured by a plurality of sensors 626 in the sensor array 616 (see FIG. 10B). The sensors 626 are coupled to a signal processor 628 that utilizes the measurement of the marker signals 622 from the sensors 626 to calculate the location of each marker assembly 614 in three-dimensional space relative to a known frame of reference, such as the sensor array 616.

Source generator 618 is configured to generate the excitation signal 620 so that one or more marker assemblies 614 are sufficiently energized to generate the marker signals 622. The source generator 618 can be switched off after the marker assemblies are energized. Once the source generator 618 is switched off, the excitation signal 620 terminates and is not measurable. Accordingly, sensors 626 in sensor array 616 will receive only marker signals 622 without any interference or magnetic field distortion induced by the excitation signal 620. Termination of the excitation signal 620 occurs before a measurement phase in which marker signals 622 are measured. Such termination of the excitation signal before the measurement phase when the energized marker assemblies 614 are generating the marker signals 622 allows for a sensor array 616 of increased sensitivity that can provide data of a high signal-to-noise ratio to the signal processor 628 for extremely accurate determination of the three-dimensional location of the marker assemblies 614 relative to the sensor array or other frame of reference.

The miniature marker assemblies 614 in the system 600 are inert, activatable assemblies that can be excited to generate a signal at a resonant frequency measurable by the sensor array 616 remote from the target on which they are placed. The miniature marker assemblies 614 have, as one example, a diameter of approximately 2 mm and a length of approximately 5 mm, although other marker assemblies can have different dimensions. An example of such a marker detection systems are described in detail in U.S. Patent Publication No. 20020193685 entitled Guided Radiation Therapy System, filed Jun. 8, 2001 and published on December 19, 2002, and U.S. Patent No. 6,822,570 to Dimmer et al., entitled System For Spacially Adjustable Excitation Of Leadless Miniature Marker, all of the disclosures of which are incorporated herein in their entirety by reference thereto.

Referring to FIG. 10C, the illustrated marker assembly 614 includes a coil 630 wound around a ferromagnetic core 632 to form an inductor (L). The inductor (L) is connected to a capacitor 634, so as to form a signal element 636. Accordingly, the signal element 636 is an inductor (L) capacitor (C) resonant circuit. The signal element 636 can be enclosed and sealed in an encapsulation member 638 made of plastic, glass, or other inert material. The illustrated marker assembly 614 is a fully contained and inert unit that can be used, as an example, in medical procedures in which the marker assembly is secured on and/or implanted in a patient's body as described in U.S. Patent No. 6,822,570 (*supra*).

The marker assembly 614 is energized, and thus activated, by the magnetic excitation field or excitation signal 620 generated by the source generator 618 such that the marker's signal element 636 generates the measurable marker signal 622. The strength of the measurable marker signal 622 is high relative to environmental background noise at the marker resonant frequency, thereby allowing the marker assembly 614 to be precisely located in three-dimensional space relative to sensor array 616.

The source generator 618 can be adjustable to generate a magnetic field 620 having a waveform that contains energy at selected frequencies that substantially match the resonant frequency of the specifically tuned marker assembly 614. When the marker assembly 614 is excited by the magnetic field 620, the signal element 636 generates the response marker signal 622 containing frequency components centered at the marker's resonant frequency. After the marker assembly 614 us energized for a selected time period, the source generator 618 is switched to the "off" position so the pulsed excitation signal 620 is terminated and provided no measurable interference with the marker signal 622 as received by the sensor array 616.

The marker assembly 614 is constructed to provide an appropriately strong and distinct signal by optimizing marker characteristics and by accurately tuning the marker assembly to a predetermined frequency. Accordingly, multiple uniquely tuned, energized marker assemblies 614 may be reliably and uniquely measured by the sensor array 616. The unique marker assemblies 614 at unique resonant frequencies may be excited and measured simultaneously or during unique time periods. The signal from the tuned miniature marker assembly 614 is significantly above environmental signal noise and sufficiently strong to allow the signal processor 628 (FIG 10A) to determine the marker assembly's identity, precise location, and orientation in three dimensional space relative to the sensor array 616 or other selected reference frame.

A system corresponding to system 600 is described in U.S. Patent No. 6,822,570 to Dimmer et al., entitled System For Spacially Adjustable Excitation Of Leadless Miniature Marker and which was filed August 7, 2002, the entire disclosure of which is hereby incorporated herein in its entirety by reference thereto. According to U.S. Patent No. 6,822,570, the system can be used in many different applications in which the miniature marker's precise three-dimensional location within an accuracy of approximately 1 mm can be uniquely identified within a relatively large navigational or excitation volume, such as a volume of 12 cm x 12cm x 12cm or greater. One such application is the use of the system to accurately track the position of targets (e.g., tissue) within the human body. In this application, the leadless marker assemblies are implanted at or near the target so the marker assemblies move with the target as a unit and provide positional references of the target relative to a reference frame outside of the body. U.S. Patent No. 6,822,570 further notes that such a system could also track relative positions of therapeutic devices (i.e., surgical tools, tissue, ablation devices, radiation delivery devices, or other medical devices) relative to the same fixed reference frame by positioning additional leadless marker assemblies on these devices at known locations or by positioning these devices relative to the reference frame. The size of the leadless markers used on therapeutic devices may be increased to allow for greater marker signal levels and a corresponding increase in navigational volume for these devices.

Other examples of leadless markers and/or devices for generating magnetic excitation fields and sensing the target signal are disclosed in U.S. Patent No. 6,889,833 to Seiler et al. and entitled Packaged Systems For Implanting Markers In A Patient And Methods For Manufacturing And Using Such Systems, U.S. Patent No. 6,812,842 to Dimmer and entitled Systems For Excitation Of Leadless Miniature Marker, U.S. Patent No. 6,838,990 to Dimmer and entitled Systems For Excitation Of Leadless Miniature Marker, U.S. Patent No. 6,977,504 to Wright et al. and entitled Receiver Used In Marker Localization Sensing System Using Coherent Detection, U.S. Patent No. 7,026,927 to Wright et al. and entitled Receiver Used In Marker Localization Sensing System And Having Dithering In Excitation, and U.S. Patent No. 6,363,940 to Krag and entitled System and Method For Bracketing And Removing Tissue all the disclosures of which are hereby incorporated herein in their entirety by reference thereto.

Another example of a suitable non-ionizing localization approach that accommodates leadless markers is the Calypso® 4D Localization System, which is a target localization platform based on detection of AC electromagnetic markers, called Beacon® transponders, which are implantable devices. These localization systems and markers have been developed by Calypso® Medical Technologies (Seattle, Washington).

In yet a further embodiment, an electromagnetic field is generated and measured external to the patient. This field is modified in a measurable manner by the stent-graft wire stent components.

Any feature described in any one embodiment described herein can be combined with any other feature of any of the other embodiments whether preferred or not.

Variations and modifications of the devices and methods disclosed herein will be readily apparent to persons skilled in the art.

## Claims

1. A method of monitoring prosthesis deployment in a patient comprising:
positioning a prosthesis delivery catheter (102), having a proximal end portion and a distal end portion, and a prosthesis (200) having a proximal end portion and a distal end portion disposed in the catheter distal end portion and at least one marker adapted to generate, respond to, or modify nonionizing energy, nonionizing radiation, or nonionizing fields, the marker (120a, b, c,...,n;122a, b, c,...,n) being on one of the prosthesis (200) and prosthesis delivery catheter (102) with the prosthesis proximal end portion at a desired location in the vasculature of the patient;
deploying the prosthesis (200) from the catheter (102); and
monitoring prosthesis movement based on the marker's (120a, b, c) generation of, response to, or modification of nonionizing energy, nonionizing radiation, or nonionizing fields.

2. The method of monitoring prosthesis deployment according to claim 1, wherein
the at least one marker is provided on the prosthesis with the prosthesis proximal end portion at a desired location in the vasculature of the patient; and further comprising
subjecting the marker to electromagnetic energy;
deploying the prosthesis from the catheter; and
monitoring prosthesis movement in real time based on a reaction that results from subjecting the at least one marker to the electromagnetic energy.

3. The method of claim 2 wherein monitoring prosthesis movement is based on a reaction that results from subjecting the marker to a plurality of electromagnetic fields.

4. The method of claim 1 or 2 wherein the reaction is the generation of signals from the marker.

5. The method of any of claims 2 to 4 wherein the at least one marker is an electromagnetic field sensing coil.

6. The method of any of claims 2 to 5 wherein the reaction is the at least one marker's modification of the electromagnetic fields.

7. The method of monitoring prosthesis deployment according to claim 1, wherein
said at least one marker is a electromagnetic field generating marker; the method further comprising
monitoring prosthesis movement based on the electromagnetic field generated by the at least one marker in response to an electromagnetic field.

8. The method of monitoring prosthesis deployment according to claim 1, wherein
said at least one marker is a non-ionizing photon radiation marker; the method further comprising
monitoring prosthesis movement based on generation of non-ionizing radiation reflected by the at least one marker an external source of radiation in the electromagnetic spectrum in the range from the infared to the ultraviolet.

9. The method of claim 8 wherein the marker generates electromagnetic spectrum radiation in the frequency of ultraviolet.

10. The method of claim 8 wherein the at least one marker generates electromagnetic spectrum radiation in the frequency of infared.

11. The method of claim 10 wherein the at least one marker is an optical LED.

12. The method of monitoring prosthesis deployment according to claim 1, wherein
said at least one marker is adapted to modify non-ionizing photon radiation; the method further comprising
subjecting the marker to non-ionizing photon radiation;
deploying the prosthesis from the catheter; and
monitoring prosthesis movement based on the at least one marker's modification of the non-ionizing photon radiation.

13. The method of claim 12 wherein the at least one marker is subjected to radio frequency radiation.

14. The method of claim 12 or 13 wherein the at least one marker is a radio frequency reflector.

15. The method of claim 12 wherein the at least one marker is an optical reflector.

16. A method of monitoring prosthesis deployment according to claim 1, wherein
said at least one marker is adapted to generate pressure waves; the method further comprising
energizing the marker with an ultrasound source;
deploying the prosthesis from the catheter; and
monitoring prosthesis movement based on pressure waves generated at the least one marker when energized.

17. The method of claim 16 wherein prosthesis movement is based on ultrasound energy generated by at least one marker when energized.

18. A prosthesis delivery system comprising:
a tubular sheath (103) having a proximal end portion and a distal end portion;
a distal tip (106) extending from the tubular sheath distal end portion;
a tubular prosthesis (200) slidably disposed in said sheath and positioned along said sheath distal end portion; and
a marker (120a, b, c,...,n; 122a, b, c,...,n) provided on one of the prosthesis (200) and the distal tip (106) and adapted to provide measurable data when subjected to non-ionizing energy.

19. The system of claim 18 wherein said marker is an electromagnetic sensing coil.

20. The system of claim 18 wherein said marker is an optical LED.

21. The system of claim 18 wherein said marker is a radio frequency beacon.

22. The system of claim 18 wherein said marker is an optical reflector.

23. The system of claim 18 wherein said marker is a radio frequency reflector.

24. The system of claim 18 wherein said marker is a pressure wave beacon.

25. The system of claim 18 wherein said marker is an ultrasound beacon.

26. The system of any of claims 18 to 25 wherein a said prosthesis has a proximal end and a distal end and said marker is positioned adjacent to said proximal end.

27. The system of any of claims 18 to 25 wherein a said prosthesis has a distal end and a generally annular proximal end and said marker is positioned along said proximal end.

28. The system of claim 26 or 27 wherein a plurality of said markers are positioned adjacent to said proximal end or along said proximal end.

29. The system of any of claims 18 to 28 wherein said prosthesis is a stent.

30. The system of any of claims 18 to 28 wherein said prosthesis is a stent-graft.
